# EUROPEAN PATENT APPLICATION

(11) **EP 3 508 183 A1**
(43) Date of publication of application: **10.07.2019**
(21) Application number: 16915105.7
(22) Date of filing: 31.08.2016
(51) Int. Cl.: A61F 13/15, A61F 13/532

(54) **DEVICE FOR MANUFACTURING ABSORBENT BODY AND METHOD FOR MANUFACTURING ABSORBENT BODY**

(71) Applicant: Unicharm Corporation, Shikokuchuo-shi, Ehime 799-0111 (JP)
(72) Inventor: TSUKUDA, Atsushi, Kanonji-shi Kagawa 769-1602 (JP); AKANO, Yukihisa, Kanonji-shi Kagawa 769-1602 (JP); TAKEUCHI, Kenji, Kanonji-shi Kagawa 769-1602 (JP)
(74) Representative: Staeger & Sperling Partnerschaftsgesellschaft mbB
(86) International application number: PCT/JP2016/075477
(87) International publication number: WO 2018/042544

(57) **Abstract**

A device for manufacturing an absorbent body includes: a shaping unit (30) that includes a recessed portion (71) and that makes liquid absorbent fiber (6) and superabsorbent polymer (SAP) to be deposited in the recessed portion (71), shaping the absorbent body (1) ; and a supply unit (20) that supplies the liquid absorbent fiber (6) and the superabsorbent polymer (SAP) to the recessed portion (71) . The recessed portion (71) includes a protruding portion (80), and the protruding portion (80) protrudes from a bottom surface and includes an inclined surface (82). In a cross section orthogonal to a ridge line of the protruding portion (80), an angle of the inclined surface (82) with respect to a surface parallel to the bottom surface is greater than an angle of repose of the superabsorbent polymer (SAP) and smaller than 90 degrees.

## Description

### [Technical Field]

The present invention relates to a method and a device for manufacturing an absorbent body.

### [Background Art]

Absorbent bodies obtained by shaping liquid absorbent fiber mixed with a superabsorbent polymer (hereinafter SAP) into a predetermined shape are known as absorbent bodies that absorb excrement and serve as one component of absorbent articles such as disposable diapers and sanitary napkins.

For example, PTL 1 discloses, as a device for manufacturing the absorbent body, a device that includes, above a belt-shaped fiber web continuously transported, a supply port of SAP (granules) extending in a width direction of the fiber web, moves a pair of guides including inclined surfaces below the supply port in the width direction to adjust a width of the supply port. Such a device can form a high-density region of the SAP on side portions of a region in which the SAP is scattered in the width direction. Then, an absorbent article having excellent absorbency can be manufactured by disposing the region in which the SAP is scattered at a high density on a portion of an absorbent body that faces an excretory portion of a wearer, for example.

### [Citation List]

### [Patent Literature]

[PTL 1] Japanese Patent Application Publication No. 2004-188285

### [Summary of Invention]

### [Technical Problem]

However, control of the guides moving in the width direction described in PTL 1 is limited and is difficult to be compatible with a fast manufacturing line of an absorbent body. Also in the device described in PTL 1, it is difficult to increase an application amount of SAP in a desired region other than the side portions in the width direction and conversely reduce an application amount of SAP in a desired region.

The present invention has been made in view of the above-mentioned problems, and an objective of the present invention is to provide a device and a method for manufacturing an absorbent body that adjust the amount of superabsorbent polymer in a desired region of the absorbent body.

### [Solution to Problem]

A main aspect of the present invention for achieving the above-described aspect is a device for manufacturing an absorbent body including a liquid absorbent fiber and a superabsorbent polymer, the device including: a shaping unit that includes a recessed portion, and that makes the liquid absorbent fiber and the superabsorbent polymer to be deposited in the recessed portion, shaping the absorbent body, the recessed portion including a protruding portion, the protruding portion protruding from a bottom surface and including an inclined surface; and a supply unit that supplies the liquid absorbent fiber and the superabsorbent polymer to the recessed portion, in a cross section orthogonal to a ridge line of the protruding portion, an angle of the inclined surface with respect to a surface parallel to the bottom surface being greater than an angle of repose of the superabsorbent polymer and smaller than 90 degrees.

Features of the present invention other than the above will become clear by reading the description of the present specification with reference to the accompanying drawings.

### [Advantageous Effects of Invention]

With the present invention, it is possible to provide a device and a method for manufacturing an absorbent body that adjust the amount of superabsorbent polymer in a desired region of the absorbent body.

### [Brief Description of the Drawings]

FIG. 1 is a schematic plan view of an absorbent body 1 manufactured by a manufacturing device according to a first embodiment.
FIG. 2 is a schematic side view of a manufacturing device 10 of the absorbent body 1 according to the first embodiment.
FIG. 3A is a schematic cross-sectional view of a shaping die 70. FIG. 3B is a schematic plan view of a side plate 72. FIG. 3C is a schematic plan view of a bottom plate 73. FIG. 3D is a schematic front view of the bottom plate 73.
FIG. 4 is a diagram for illustrating vent holes 76 of the bottom plate 73.
FIGS. 5A and 5B are diagrams illustrating a state where pulp fiber 6 and SAP are deposited on the bottom plate 73. FIG. 5C is a diagram illustrating regions A1 to A3 with basis weights of the SAP different from each other.
FIGS. 6A and 6B are diagrams illustrating modified examples of a protruding portion 80.
FIGS. 7A and 7B are diagrams for illustrating a manufacturing device of an absorbent body 1 according to a second embodiment.
FIGS. 8A and 8B are diagrams for illustrating a recessed portion 71 of a manufacturing device according to a third embodiment.
FIGS. 9A and 9B are diagrams for illustrating a different recessed portion 71 of the manufacturing device according to the third embodiment.

### [Description of Embodiments]

At least the following matters will become clear with the description of this specification and the attached drawings.

A device for manufacturing an absorbent body including a liquid absorbent fiber and a superabsorbent polymer, the device including:
a shaping unit
   that includes a recessed portion, and
   that makes the liquid absorbent fiber and the superabsorbent polymer to be deposited in the recessed portion, shaping the absorbent body,
   the recessed portion including a protruding portion,
   the protruding portion protruding from a bottom surface and including an inclined surface; and
a supply unit that supplies the liquid absorbent fiber and the superabsorbent polymer to the recessed portion,
in a cross section orthogonal to a ridge line of the protruding portion,
   an angle of the inclined surface with respect to a surface parallel to the bottom surface being greater than an angle of repose of the superabsorbent polymer and smaller than 90 degrees.

According to the device for manufacturing an absorbent body, while the liquid absorbent fiber is deposited on the inclined surface of the protruding portion, the superabsorbent polymer rolls down on the inclined surface and is deposited on a boundary portion between the bottom surface of the recessed portion and the inclined surface. This decreases the amount of the superabsorbent polymer included in the liquid absorbent fiber deposited on the inclined surface, and this increases the amount of the superabsorbent polymer included in the liquid absorbent fiber deposited on the boundary portion between the bottom surface of the recessed portion and the inclined surface. In other words, the amount of the superabsorbent polymer in a desired region of the absorbent body can be adjusted by adjusting the position of the inclined surface of the protruding portion provided on the recessed portion.

In such a device for manufacturing an absorbent body,
the protruding portion includes a second inclined surface, and
in the cross section,
an angle of the second inclined surface with respect to a surface parallel to the bottom surface is greater than the angle of repose of the superabsorbent polymer and smaller than 90 degrees, and
the angle of the second inclined surface is different from the angle of the inclined surface.

According to the device for manufacturing an absorbent body, the superabsorbent polymer rolls down to both sides in a direction orthogonal to the ridge line of the protruding portion. Thus, a region in which the amount of the superabsorbent polymer is increased can be formed on both sides of a region in which the amount of the superabsorbent polymer decreases by the inclined surface. Furthermore, an increase amount of the superabsorbent polymer can be set to be different by setting a different angle of the inclined surfaces.

In such a device for manufacturing an absorbent body,
the protruding portion includes a second inclined surface, and
a flat surface is not provided between the inclined surface and the second inclined surface.

According to the device for manufacturing an absorbent body, deposition of the superabsorbent polymer at the center of a region in which the amount of the superabsorbent polymer decreases by the inclined surface can be suppressed.

In such a device for manufacturing an absorbent body,
the supply unit supplies the liquid absorbent fiber and the superabsorbent polymer to the recessed portion moving in a predetermined direction, and
a ridge line of the protruding portion includes a curved portion curved with respect to the predetermined direction.

According to the device for manufacturing an absorbent body, the superabsorbent polymer rolling down the inclined surface and being deposited is dispersed through a direction orthogonal to the predetermined direction. Thus, even when the superabsorbent polymer moves upstream in the predetermined direction by inertial force, it is possible to suppress concentration of the superabsorbent polymer at a certain part in the direction orthogonal to the predetermined direction.

In such a device for manufacturing an absorbent body,
the shaping unit makes the liquid absorbent fiber and the superabsorbent polymer to be deposited in the recessed portion by suction,
the bottom surface and the protruding portion are formed of an air-permeable member having a plurality of vent holes, and
the vent holes in the air-permeable member have a uniform aperture ratio.

According to the device for manufacturing an absorbent body, the basis weight of the superabsorbent polymer can be adjusted using the inclined surface of the protruding portion while the basis weight of the liquid absorbent fiber is uniform over the entire region of the absorbent body.

In such a device for manufacturing an absorbent body,
the shaping unit makes the liquid absorbent fiber and the superabsorbent polymer to be deposited in the recessed portion by suction,
the bottom surface and the protruding portion are formed of an air-permeable member having a plurality of vent holes, and
an aperture ratio of the vent holes is different depending on a portion of the air-permeable member.

According to the device for manufacturing an absorbent body, the basis weight of the superabsorbent polymer can be adjusted using the inclined surface of the protruding portion while the basis weight of the liquid absorbent fiber is adjusted using the aperture ratio of the air-permeable member.

In such a device for manufacturing an absorbent body,
a reinforcing member along the air-permeable member is provided inside the air-permeable member,
the reinforcing member includes a plurality of second vent holes that is larger than the vent holes, and
the second vent hole disposed in a portion of the reinforcing member corresponding to the inclined surface are smaller than the second vent hole disposed in a portion of the reinforcing member corresponding to the bottom surface.

According to the device for manufacturing an absorbent body, it is possible to reduce a loss of suction force caused by addition of the reinforcing member while strength of the air-permeable member is increased. Further, the suction force acting on the air-permeable member depends on the aperture ratio of the air-permeable member, facilitating adjustment of the basis weight of the liquid absorbent fiber. In addition, the rigidity of the protruding portion increases, making it possible to suppress deformation of and damage to the air-permeable member and the reinforcing member in the protruding portion.

In such a device for manufacturing an absorbent body,
a negative pressure in a space communicating with the vent hole is different depending on a portion of the air-permeable member.

According to the device for manufacturing an absorbent body, adjusting the negative pressure of the space communicating with the vent hole makes it possible to adjust the basis weight of the superabsorbent polymer using the inclined surface of the protruding portion while adjusting the basis weight of the liquid absorbent fiber.

In such a device for manufacturing an absorbent body,
the device further comprises a compressing unit that compresses, in a thickness direction of the absorbent body, a portion of the absorbent body formed using the inclined surface.

According to the device for manufacturing an absorbent body, compression can be properly performed on a region in which the amount of the superabsorbent polymer decreases due to the inclined surface of the protruding portion. Further, movement of the liquid absorbent fiber and the superabsorbent polymer is restricted as a result of compression, and therefore there maintains the distribution of the liquid absorbent fiber and the superabsorbent polymer, which has been adjusted using the inclined surface of the protruding portion and the like. In addition, it is possible to prevent the absorbent body from excessively hardening due to the compression of the superabsorbent polymer.

In such a device for manufacturing an absorbent body,
the device further comprises a second compressing unit that compresses, in the thickness direction of the absorbent body, a portion of the absorbent body including at least a part of a portion formed of the bottom surface.

According to the device for manufacturing an absorbent body, movement of the liquid absorbent fiber and the superabsorbent polymer is restricted due to compression, and this maintains the distribution of the liquid absorbent fiber and the superabsorbent polymer, which has been adjusted by the inclined surface of the protruding portion and the like.

In such a device for manufacturing an absorbent body,
the supply unit supplies the liquid absorbent fiber and the superabsorbent polymer to the recessed portion moving in a predetermined direction, and
a side surface of the recessed portion on an upstream side in the predetermined direction is an inclined surface or a step surface having a step,
the inclined surface and the step surface being inclined toward the upstream side outwardly from the bottom surface.

According to the device for manufacturing an absorbent body, even when the superabsorbent polymer moves upstream in the predetermined direction due to inertial force, the superabsorbent polymer is more likely to jump outside the recessed portion because of the inclined surface or the step surface. This makes it possible to suppress concentration of the superabsorbent polymer near the side surface on the upstream side of the recessed portion.

In such a device for manufacturing an absorbent body,
the supply unit supplies the liquid absorbent fiber and the superabsorbent polymer to the recessed portion moving in a predetermined direction, and
a concave/convex portion having a height lower than the inclined surface and elongating in the predetermined direction is provided on a portion of the bottom surface around the inclined surface.

According to the device for manufacturing an absorbent body, the liquid absorbent fiber is deposited in conformance with the shape of the concave/convex portion. Thus, movement of the superabsorbent polymer toward upstream due to inertial force is restricted by a protruding portion of the liquid absorbent fiber deposited in an undulating manner. This makes it possible to suppress concentration of the superabsorbent polymer near the side surface on the upstream side of the recessed portion.

A method for manufacturing an absorbent body including a liquid absorbent fiber and a superabsorbent polymer, the method including:
supplying the liquid absorbent fiber and the superabsorbent polymer to a recessed portion that is for shaping the absorbent body; and
shaping the absorbent body by depositing the liquid absorbent fiber and the superabsorbent polymer in the recessed portion,
   the recessed portion including a protruding portion,
   the protruding portion protruding from a bottom surface and including an inclined surface,
   in a section orthogonal to a ridge line of the protruding portion,
      an angle of the inclined surface with respect to a surface parallel to the bottom surface being greater than an angle of repose of the superabsorbent polymer and smaller than 90 degrees.

According to the method for manufacturing an absorbent body, while the liquid absorbent fiber is deposited on the inclined surface of the protruding portion, the superabsorbent polymer rolls down on the inclined surface and is deposited on a boundary portion between the bottom surface of the recessed portion and the inclined surface. This decreases the amount of the superabsorbent polymer included in the liquid absorbent fiber deposited on the inclined surface, and this increases the amount of the superabsorbent polymer included in the liquid absorbent fiber deposited on the boundary portion between the bottom surface of the recessed portion and the inclined surface. In other words, the amount of the superabsorbent polymer in a desired region of the absorbent body can be adjusted by adjusting the position of the inclined surface of the protruding portion provided on the recessed portion.

### First Embodiment

### Configuration of Absorbent Body 1:

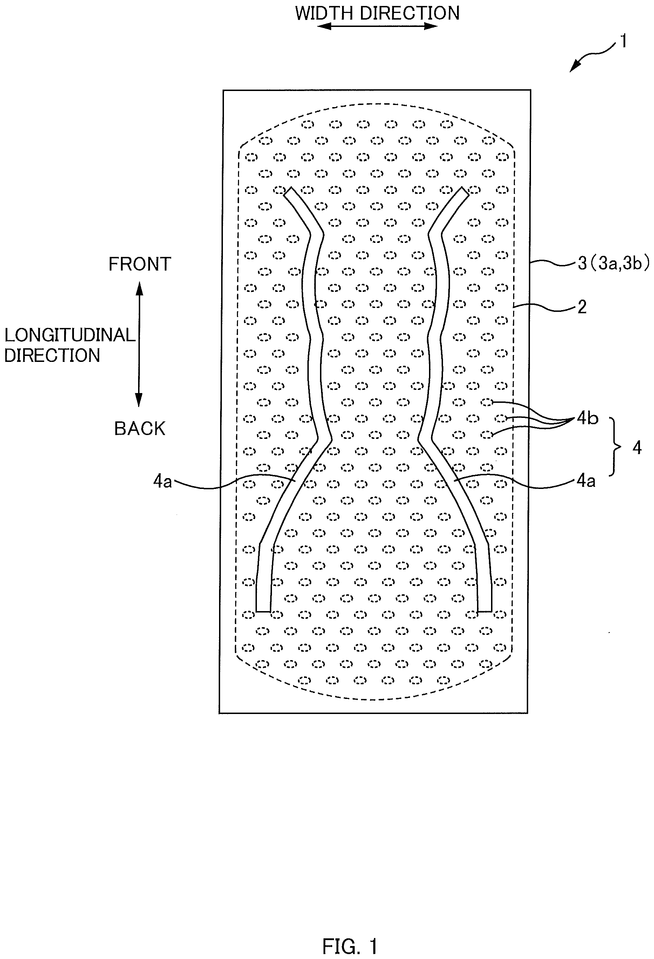

FIG. 1 is a schematic plan view of an absorbent body 1 manufactured by a manufacturing device according to a first embodiment. The absorbent body 1 is one component of an absorbent article such as a disposable diaper, a sanitary napkin, and a urine absorbing pad, and the absorbent body 1has a function of absorbing excrement such as urine and menstrual blood. The absorbent body 1 has a rectangle planar shape, and has a longitudinal direction, a width direction, and a thickness direction orthogonal to one another.

The absorbent body 1 includes an absorbent core 2 that absorbs liquid and a core wrapping sheet 3 that covers an outer circumferential surface of the absorbent core 2. The absorbent core 2 is obtained by shaping a liquid absorbent fiber containing a superabsorbent polymer into a predetermined shape. The absorbent core 2 in the present embodiment is shaped into a substantially rectangular shape with curved ends in the longitudinal direction. Further, the absorbent core 2 is sandwiched between two rectangular core wrapping sheets 3a and 3b. However, the shape of the absorbent core 2 is not limited to that described above, and may be a substantially hourglass shape in which the longitudinal central portion is constricted inwardly in the width direction, for example. Alternatively, one core wrapping sheet 3 may wrap the absorbent core 2, or the absorbent core 2 alone may be the absorbent body 1 without the core wrapping sheet 3.

The liquid absorbent fiber can be exemplified by cellulosic short fiber (pulp fiber, rayon fiber, and cotton fiber), synthetic short fiber (polyethylene), and any combination thereof.

A superabsorbent polymer has a particulate shape and a particle size of about 50 to 1000 µm. The superabsorbent polymer can be exemplified by starch-acrylic (salt) graft copolymer, saponified starch-acrylonitrile copolymer, sodium carboxymethylcellulose cross-linked material, and acrylic acid (salt) polymer, and any combination thereof.

Examples of the core wrapping sheet 3 include a liquid permeable sheet such as tissue paper and a nonwoven fabric.

A compressed portion 4 being recessed in the thickness direction by embossing (compressing processing) is formed in the absorbent body 1. The compressed portion 4 includes linear compressed portions 4a and spot-like compressed portions 4b. The linear compressed portions 4a are a pair of linear recessed portions extending in the longitudinal direction and provided at both widthwise side portions of the absorbent body 1. Note that, the linear compressed portions 4a may be formed by compressing its entire region into a groove shape or formed by compression in a discrete manner. The spot-like compressed portions 4b are many elliptical recessed portions arranged in a staggered pattern over the entire surface of the absorbent body 1. In the present embodiment, the linear compressed portions 4a are formed by stronger compression compared to the spot-like compressed portions 4b, and have a greater depth of the recessed portion. Note that, concerning the compressed portions 4, their arrangement, shape, size and the like are not limited to those illustrated in FIG. 1, and may be appropriately set according to the use.

Providing the compressed portions 4 in the absorbent body 1 can increase rigidity of the absorbent body 1, and can restrict movement of the liquid absorbent fiber and the superabsorbent polymer constituting the absorbent core 2. This makes it possible to prevent the absorbent body 1 from losing its shape. In particular, providing the spot-like compressed portions 4b over a large plane range of the absorbent body 1 can increase the effect.

Furthermore, providing the pair of linear compressed portions 4a in the absorbent body 1 makes it easier for the absorbent body 1 to change its shape to fit the wearer. For example, the linear compressed portions 4a in the present embodiment each have the longitudinal central portion being constricted inwardly in the width direction. This makes it easier for the longitudinal central portion of the absorbent body 1 to bend from the linear compressed portions 4a by being sandwiched between the legs of the wearer. And, the longitudinal central portion easily deform into a cup shape that covers a crotch portion of the wearer. The linear compressed portions 4a spread outward in the width direction longitudinally backward (back side of the wearer). Accordingly, the linear compressed portions 4a increases the rigidity of a longitudinal back portion of the absorbent body 1, making it easier to maintain a surface shape along the buttock of the wearer.

It is assumed that, in the absorbent body 1 in the first embodiment, the basis weight (g/m²) of liquid absorbent fiber (hereinafter also referred to as "pulp fiber") is uniform over the entire region of the absorbent body 1. Supposing that an excessive amount of super absorbent polymer (hereinafter also referred to as "SAP") is disposed in a relatively-strongly-compressed portion like the linear compressed portions 4a, there is a risk of causing insufficient compression of the pulp fiber and excessive hardening of the linear compressed portions 4a, making less comfortable fit of an absorbent article. Accordingly, when the SAP is scattered evenly over the entire region of the absorbent body 1 similarly to the pulp fiber, the problem described above occurs.

Thus, in the absorbent body 1 in the first embodiment, while the basis weight of the pulp fiber is uniform over the entire region of the absorbent body 1, the linear compressed portions 4a have a smaller basis weight (g/m²) of the SAP than that in other portions. In other words, the ratio of the SAP is reduced with respect to the pulp fiber having a uniform basis weight. A device and a method for manufacturing the absorbent body 1 will be described below.

### Basic Configuration of Manufacturing device 10 of Absorbent Body 1:

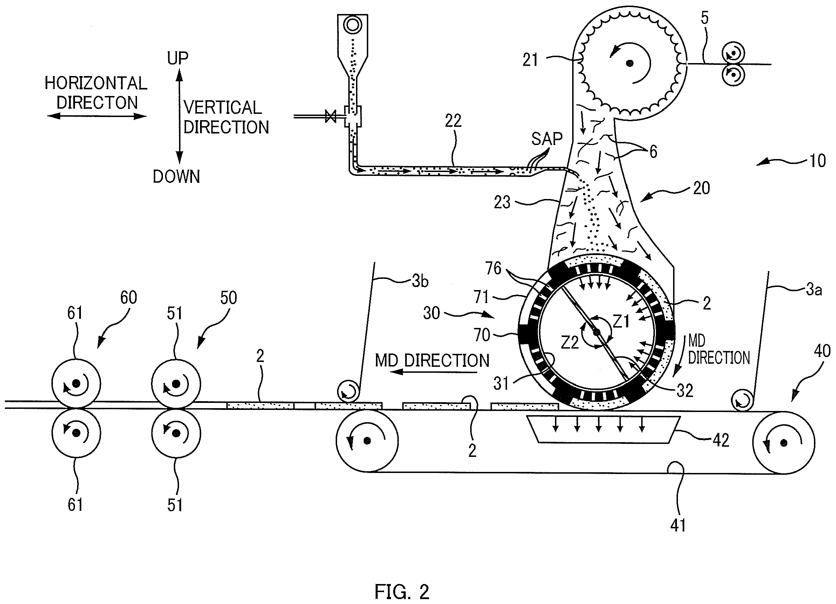

FIG. 2 is a schematic side view of a manufacturing device 10 of the absorbent body 1 according to the first embodiment.

The manufacturing device 10 of the absorbent body 1 includes: a "supply unit 20" that supplies a pulp fiber 6 and the SAP to a recessed portion 71 of a shaping die 70; a "shaping unit 30" in which the pulp fiber 6 and the SAP are deposited into the recessed portion 71 by suction, shaping the absorbent core 2 (absorbent body 1); a "suction belt conveyer 40" that releases the absorbent body 1 from the shaping unit 30; an "upstream embossing unit 50" that forms the spot-like compressed portions 4b in the absorbent body 1; and a "downstream embossing unit 60" that forms the pair of linear compressed portions 4a in the absorbent body 1.

The supply unit 20 includes a pulverizer 21 that pulverizes a pulp sheet 5 into the pulp fiber 6, SAP supply tube 22, and a duct 23 that guides the pulp fiber 6 and the SAP to the shaping unit 30. The duct 23, whose tube axial direction along the vertical direction, has an upper opening that communicates with the pulverizer 21, and has a lower opening that covers the top portion of the outer circumferential surface of a rotary drum 31 described later. The SAP supply tube 22 has a tip portion inserted into the duct 23.

The shaping unit 30 includes the rotary drum 31 having a hollow cylindrical shape and the recessed die 70 provided along the outer circumferential surface of the rotary drum 31. A plurality of recessed portions 71 (six recessed portions 71 in FIG. 2) are formed intermittently on the recessed die 70 in a circumferential direction of the recessed die 70. The planar shape of the recessed portion 71 corresponds to the external shape of the absorbent core 2. The shaping die 70 rotates together with the rotary drum 31. Therefore, the supply unit 20 (duct 23) supplies the pulp fiber 6 and the SAP to the recessed portions 71 moving in the circumferential direction (predetermined direction) of the rotary drum 31.

Note that, in the present embodiment, the circumferential direction of the rotary drum 31 and the direction of transport (MD direction) in the manufacturing device 10 correspond to the longitudinal direction of the absorbent body 1. Further, the rotation axis direction of the rotary drum 31 and the CD direction orthogonal to the MD direction correspond to the width direction of the absorbent body 1. However, the directions are not limited to these, and the width direction of the absorbent body 1 may correspond to the MD direction. In the present embodiment, the recessed portions 71 are moved by the rotary drum 31, but the recessed portions 71 may be moved by a belt conveyer and the like, for example.

Many vent holes 76 are formed in the bottom portion of each recessed portion 71. Space in the recessed portion 71 communicates with space on an inner circumferential side of the rotary drum 31 through the vent holes 76. A partition wall 32 fixed regardless the rotation of the rotary drum 31 is provided in the space on the inner circumferential side of the rotary drum 31. The space on the inner circumferential side of the rotary drum 31 is divided into a first zone Z1 and a second zone Z2 by the partition wall 32. The first zone Z1 is in a negative pressure state by a suction mechanism (not shown), and the second zone Z2 is under atmospheric pressure. Thus, suction is performed from the vent holes 76 when the recessed portion 71 passes through the first zone Z1, and suction is not performed from the vent holes 76 when the recessed portion 71 passes through the second zone Z2. Note that, it is assumed in the first embodiment that the suction force is constant in the first zone Z1.

The opening of the duct 23 faces the first zone Z1 of the rotary drum 31 and covers the outer circumferential surface of the rotary drum 31 over a range wider than one of the recessed portions 71. Thus, in the duct 23, air flow is generated by suction from the vent holes 76 of the recessed portion 71. Accordingly, the pulp fiber 6 pulverized by the pulverizer 21 and the SAP supplied from the SAP supply tube 22 are supplied to the recessed portion 71 by the air flow, deposited in the recessed portion 71, and shaped into the shape of the absorbent core 2. Note that, it is assumed that constant amounts of the pulp fiber 6 and the SAP are continuously supplied from the duct 23.

The suction belt conveyer 40 includes: a transport belt 41 with vent holes (not shown); and a suction mechanism 42. The suction mechanism 42 faces the second zone Z2 of the rotary drum 31 with the transport belt 41 therebetween. Thus, the absorbent core 2 in each recessed portion 71 is released from the recessed portion 71 by suction of the suction mechanism 42 and transferred onto the transport belt 41. Subsequently, the absorbent core 2 is transported to the upstream embossing unit 50.

In the absorbent body 1, the absorbent core 2 is sandwiched between the two core wrapping sheets 3a and 3b. Thus, the core wrapping sheet 3a (continuous sheet) is supplied to the suction belt conveyer 40 at a position upstream of the rotary drum 31, and the absorbent core 2 is placed on the core wrapping sheet 3a. Then, the different core wrapping sheet 3b (continuous sheet) is supplied at a position downstream of the rotary drum 31 on the absorbent core 2, and the absorbent core 2 is thus sandwiched between the two core wrapping sheets 3a and 3b. Note that, although it is not shown, it is preferable that the absorbent core 2 and the two core wrapping sheets 3a and 3b are joined integrally by an adhesive.

The upstream embossing unit 50 and the downstream embossing unit 60 respectively include a pair of upper and lower rolls 51 and a pair of upper and lower rolls 61 each having a rotation axis direction along the CD direction. The outer circumferential surface of one of the pair of the rolls 51 of the upstream embossing unit 50 has projections (not shown) corresponding to the spot-like compressed portions 4b formed thereon, and the other roll 51 has a flat outer circumferential surface. Similarly, the outer circumferential surface of one of the pair of the rolls 61 of the downstream embossing unit 60 has projections (not shown) corresponding to the pair of linear compressed portions 4a formed thereon, and the other roll 61 has a flat outer circumferential surface.

When the absorbent core 2 sandwiched between the two core wrapping sheets 3a and 3b passes between the pair of rolls 51 of the upstream embossing unit 50, a part of the absorbent core 2 is compressed in the thickness direction by the projections of the roll 51, forming the spot-like compressed portions 4b. Similarly, when the absorbent core 2 passes between the pair of rolls 61 of the downstream embossing unit 60, a part of the absorbent core 2 is compressed in the thickness direction by the projections of the roll 61, forming the pair of linear compressed portions 4a. The compressed portion 4 thus formed allows the absorbent core 2 and the two core wrapping sheets 3a and 3b to be joined integrally. Subsequently, although it is not shown, the core wrapping sheets 3a and 3b being continuous sheets are cut into a product size, and the absorbent body 1 (FIG. 1) in the first embodiment is manufactured.

Note that, compression with heat may be performed by heating at least one roll of each pair of the upper and lower rolls 51 and 61, or compression may be performed by ultrasonic vibration. In order to perform compression for the linear compressed portions 4a more strongly than compression for the spot-like compressed portions 4b, it is preferable to increase pressure in the thickness direction applied to the absorbent core 2 during compression, or a temperature of the rolls 61. For general absorbent articles, a liquid-permeable sheet is disposed on a skin side of the absorbent body 1, and a liquid-impermeable sheet is disposed on a non-skin side of the absorbent body 1. The absorbent body 1 may be compressed together with these sheets.

### Shaping Die 70:

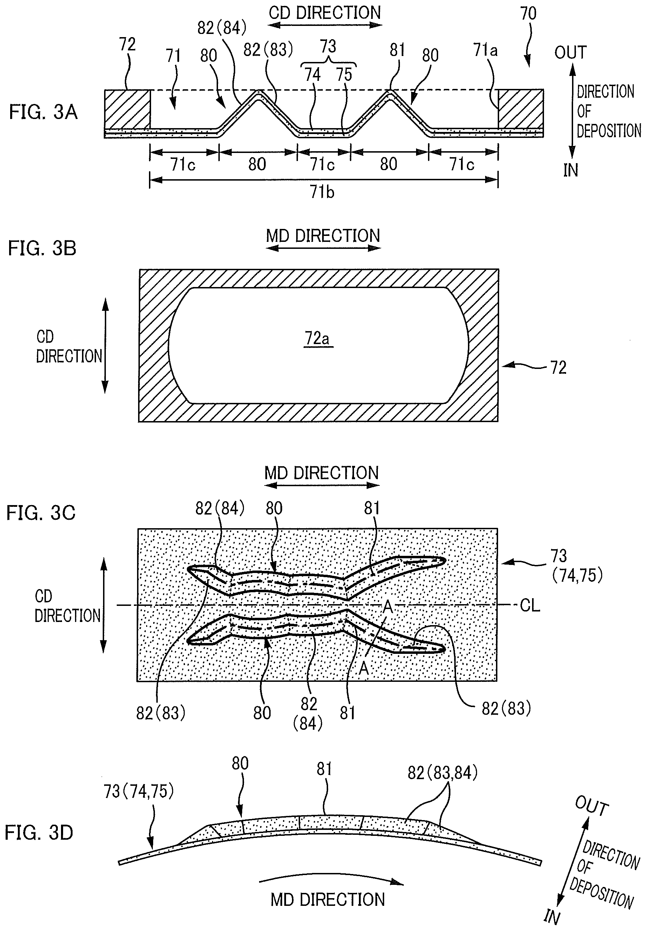

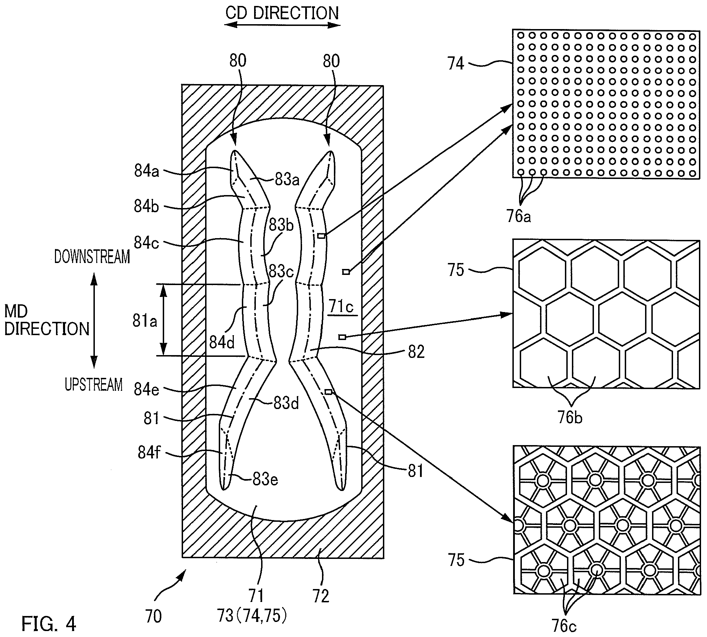

FIG. 3A is a schematic cross-sectional view of the shaping die 70 taken along the CD direction. FIG. 3B is a schematic plan view of a side plate 72. FIG. 3C is a schematic plan view of a bottom plate 73. FIG. 3D is a schematic front view of the bottom plate 73 as viewed in the CD direction. FIG. 4 is a diagram illustrating the vent holes 76 of the bottom plate 73. A schematic plan view of the shaping die 70 is illustrated on the left of FIG. 4. An enlarged view of vent holes 76a to 76c of an air-permeable member 74 and a reinforcing member 75 is illustrated on the right of FIG. 4.

FIGS. 3B to 3D illustrate a part of the shaping die 70 provided along the outer circumferential surface of the rotary drum 31. Specifically, FIGS. 3B to 3D illustrate a part of the side plate 72 and the bottom plate 73 forming one of the plurality of recessed portions 71 of the shaping die 70. In the following description, the thickness direction of the absorbent core 2 and a depth direction of the recessed portions 71 corresponding to a radial direction of the rotary drum 31 are also referred to as a "direction of deposition".

As illustrated in FIG. 3A, the shaping die 70 includes the "side plate 72" that forms a side portion 71a of the recessed portion 71 and the "bottom plate 73" that forms a bottom portion 71b of the recessed portion 71. The side plate 72 is placed on the bottom plate 73, and thus the recessed portion 71 that shapes the pulp fiber 6 and the SAP is formed.

As illustrated in FIG. 3B, the side plate 72 has an opening 72a that penetrates in the direction of deposition. The peripheral shape of the opening 72 corresponds to the external shape of the absorbent core 2.

The bottom plate 73 includes the "air-permeable member 74" and the "reinforcing member 75" that overlays the air-permeable member 74 from the inside (rotary drum 31 side) in the direction of deposition. The bottom plate 73 has a size greater than a size of the opening 72a of the side plate 72 as seen from the direction of deposition. Thus, the opening 72a is covered with the bottom plate 73, forming the bottom portion 71b of the recessed portion 71.

Note that, the side plate 72 and the bottom plate 73 may be attached to the rotary drum 31 while being integrally joined by welding. Or the bottom plate 73 and the side plate 72 may be installed and fixed in this order to the rotary drum 31 with a bolt and the like. A material for the side plate 72 and the bottom plate 73 is exemplified by a metal plate, such as a stainless steel plate (SUS304), and a resin plate.

As illustrated in FIG. 3C, the bottom plate 73 includes a "pair of protruding portions 80" that protrudes from the bottom surface of the recessed portion 71 to the outside (supply unit 20 side) in the direction of deposition. The pair of protruding portions 80 are provided in both CD-direction side portions of the bottom plate 73 with extending in the MD direction, and have a symmetrical shape with respect to a central line CL in the CD direction.

As illustrated in FIG. 3A, each of the protruding portions 80 has a mountain-shaped cross section and includes two inclined surfaces 82. Specifically, the protruding portion 80 includes: an inner inclined surface 83 inclined downward inwardly in the CD direction with respect to a ridge line 81 (top portion of the protruding portion 80) extending in the MD direction; and an outer inclined surface 84 inclined downward outwardly in the CD direction with respect to the ridge line 81.

The ridge lines 81 (dot-and-dash lines in the drawings) of the protruding portions 80 are curved lines corresponding to the shape of the linear compressed portions 4a. The inclined surfaces 82 of the protruding portions 81 are disposed in a position where the pair of linear compressed portions 4a is formed in the absorbent core 2 which is deposited on the bottom plate 73. Specifically, the ridge lines 81 of the protruding portions 80 have both MD-direction end portions extending outward in the CD direction, and have a plurality of curved portions (for example 81a illustrated in FIG. 4) protruding outward in the CD direction. Thus, the inner inclined surface 83 and the outer inclined surface 84 of each protruding portion 81 are formed by a plurality of inclined surfaces (83a to 83e and 84a to 84f illustrated in FIG. 4, respectively) being connected in the MD direction.

The reinforcing member 75 that overlays the air-permeable member 74 in the bottom plate 73 has a shape in conformance with the air-permeable member 74. Thus, as illustrated in FIG. 3A, a part of not only the air-permeable member 74 but also the reinforcing member 75 protrudes as the protruding portions 80. The protruding portions 80 have a height substantially identical to a depth of the recessed portion 71. In the following description, a substantially horizontal surface (actually, a surface curved in the MD direction along the outer periphery of the rotary drum 31) other than the protruding portions 80 in the bottom plate 73 is referred to as a "bottom surface 71c of the recessed portion 71" (corresponding to a bottom surface in the present invention) . The bottom surface 71c of the recessed portion 71 and the protruding portions 80 are collectively referred to as "the bottom portion 71b of the recessed portion".

As illustrated in FIG. 4, the air-permeable member 74 and the reinforcing member 75 forming the bottom plate 73 each have the vent holes 76 penetrating in the thickness direction thereof. Vent holes 76a of the air-permeable member 74 are holes having a size that does not allow the pulp fiber 6 and the SAP to pass through the holes. Vent holes 76b and 76c of the reinforcing member 75 are holes larger than the vent holes 76a of the air-permeable member 74. The vent holes 76a of the air-permeable member 74 and the vent holes 76b and 76c of the reinforcing member 75 communicate with each other. The space in the recessed portion 71 communicates with the space on the inner circumferential side of the rotary drum 31 through these vent holes 76a to 76c. Thus, as described above, suction is performed from the vent holes 76 when the recessed portion 71 passes through the first zone Z1 of the rotary drum 31.

The vent holes 76a to 76c are formed in not only the bottom surface 71c of the recessed portion 71 but also in the protruding portions 80, and suction is also performed in the protruding portions 80. Note that, the side portion 71a of the recessed portion 71 may be provided with vent holes that communicate with the space on the inner circumferential side of the rotary drum 31, and suction may be performed. The shape (such as a circle and a hexagon) of the vent holes 76 illustrated in FIG. 4 is one example, and the shape is not limited to this example.

The bottom plate 73 as described above is manufactured as follows, for example. First, the vent holes 76 are formed in each of a flat plate member to be the air-permeable member 74 and a flat plate member to be the reinforcing member 75. The forming of the vent holes 76 may be performed by a known method, such as laser processing, etching processing, and punching processing. Next, the plate member to be the air-permeable member 74 and the plate member to be the reinforcing member 75 are joined together. The joining may be performed by a known method, such as diffusion joining and welding. Pressing is performed on the flat plate members joined integrally in such a manner, forming the protruding portions 80. Making the flat plate members curved in the MD direction along the outer circumferential surface of the rotary drum 31, manufacturing the bottom plate 73.

As described above, in the manufacturing device 10 of the absorbent body 1 in the first embodiment, the reinforcing member 75 along the air-permeable member 74 is provided inside the air-permeable member 74. This increases strength of the air-permeable member 74. This enables the air-permeable member 74 to resist the suction force from the rotary drum 31, and this makes it possible to suppress deformation of and damage to the air-permeable member 74. However, it is not necessary to include the reinforcing member 75.

In the reinforcing member 75, disposed are the plurality of vent holes 76b and 76c (second vent holes) that are larger than the vent holes 76a of the air-permeable member 74. This makes it possible to reduce a loss of the suction force even with the reinforcing member 75, making the suction efficient. Further, such larger vent holes 76b and 76c of the reinforcing member 75 result in that the suction force acting on the bottom portion 71b of the recessed portion 71 depends on the aperture ratio of the air-permeable member 74. This makes it easier to adjust the suction force, facilitating adjustment of the basis weight of the pulp fiber 6. The pulp fiber 6 and the SAP remain in the air-permeable member 74, and therefore a problem of a missing member does not occur even when the vent holes 76b and 76c of the reinforcing member 75 are larger.

However, the protruding portions 80 formed by pressing or the like have a strength lower than that of the bottom surface 71c of the recessed portion 71, and are more likely to be deformed. Accordingly, as illustrated in FIG. 4, the vent holes 76c disposed in a portion of the reinforcing member 75 corresponding to the inclined surfaces 82 of the protruding portions 80 is preferably smaller than the vent holes 76b disposed in a portion of the reinforcing member 75 corresponding to the bottom surface 71c of the recessed portion 71. This increases the rigidity of the protruding portions 80 in the reinforcing member 75, making it possible for the protruding portions 80 to resist the suction force similarly to the bottom surface 71c of the recessed portion 71. It is possible to suppress deformation of and damage to the air-permeable member 74 and the reinforcing member 75 in the protruding portions 80. Further, it is also possible to suppress damage to members caused when the protruding portions 80 are formed by pressing or the like.

If the bottom surface 71c of the recessed portion 71 and the protruding portions 80 are formed of separated members, the pulp fiber 6 and the SAP are more likely to get caught in joints therebetween. In contrast, the protruding portions 80 are formed by pressing on the plate members in the bottom plate 73 in the present embodiment, and the bottom surface 71c of the recessed portion 71 and the protruding portions 80 are formed of one identical member as a single unit. Thus, the pulp fiber 6 and the SAP are less likely to get caught in boundary portions between the bottom surface 71c of the recessed portion 71 and the protruding portions 80. This facilitates maintenance, and this makes it easier for the absorbent core 2 to be released from the recessed portion 71. As illustrated in FIG. 3A, the bottom surface 71c of the recessed portion 71 is not located below the protruding portions 80, making it possible to reduce a loss of the suction force, and making suction efficient.

### Adjustment of Basis Weight of Pulp Fiber and SAP:

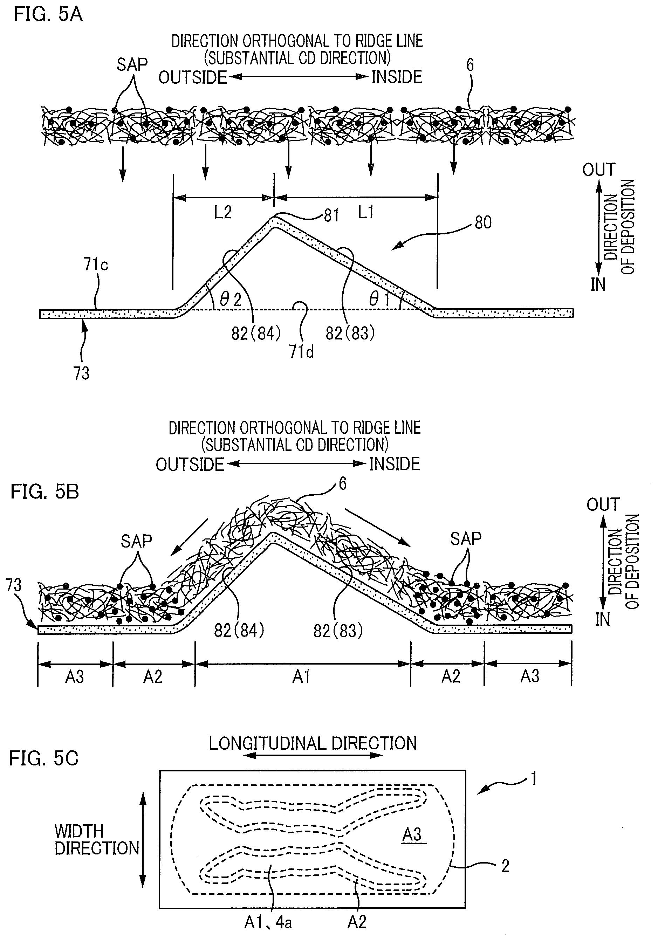

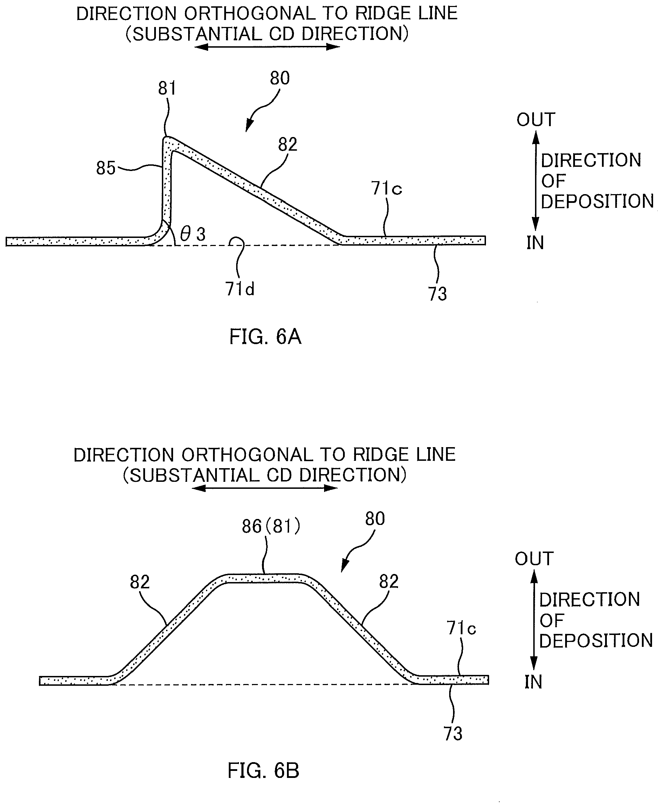

FIGS. 5A and 5B are diagrams illustrating a state where the pulp fiber 6 and the SAP are deposited on the bottom plate 73. FIG. 5C is a diagram illustrating regions A1 to A3 with basis weights of the SAP different from each other in the absorbent body 1. A cross section of the bottom plate 73 illustrated in FIGS. 5A and 5B is a cross section taken along a direction orthogonal to the ridge line 81 of the protruding portion 80, for example, a cross section taken along line A-A in FIG. 3C. FIGS. 6A and 6B are diagrams illustrating a modified example of the protruding portion 80.

As described above, the recessed portion 71 for shaping the absorbent core 2 includes the protruding portion 80 protruding from the bottom surface 71c of the recessed portion 71 and including the inclined surfaces 82. In the cross section (FIG. 5A) orthogonal to the ridge line 81 of the protruding portion 80, the angles θ1 and θ2 of the inclined surfaces 82 with respect to a surface (for example, item 71d in FIG. 5A) parallel to the bottom surface 71c of the recessed portion 71 are smaller than 90 degrees and greater than the angle of repose of the superabsorbent polymer (SAP) supplied to the recessed portion 71.

Thus, when the duct 23 located above the rotary drum 31 supplies the SAP from vertically above toward the recessed portion 71 as illustrated in FIG. 2, the SAP reaching the inclined surfaces 82 of the protruding portion 80 rolls down on the inclined surfaces 82 or on the pulp fiber 6 deposited on the inclined surfaces 82 whereas the SAP reaching the bottom surface 71c of the recessed portion 71 relatively has a tendency to stay. Therefore, even when a constant amount of the SAP is continuously supplied from the duct 23 to the recessed portion 71, the amount of the SAP included in the pulp fiber 6 deposited on the inclined surfaces 82 (region A1) is smaller than the amount of the SAP included in the pulp fiber 6 deposited on the bottom surface 71c (region A3), as illustrated in FIG. 5B. In addition, the amount of the SAP included in the pulp fiber 6 deposited on the boundary portions (region A2) between the inclined surfaces 82 and the bottom surface 71c is greater than the amount of the SAP included in the pulp fiber 6 deposited on the bottom surface 71c (region A3) as also illustrated in FIG. 5B.

Accordingly, a basis weight of the SAP in a desired region of the absorbent body 1 can be adjusted by adjusting the position of the inclined surfaces 82 of the protruding portion 80 provided on the recessed portion 71. Specifically, concerning a portion of the absorbent core 2 in which the basis weight of the SAP is desired to decrease, it is preferable that the corresponding-to-the-portion portion of the bottom portion 71b of the recessed portion 71 has the inclined surfaces 82 of the protruding portion 80. Conversely, concerning a portion of the absorbent core 2 in which the basis weight of the SAP is desired to increase, it is preferable that, around the corresponding-to-the-portion portion of the bottom portion 71b of the recessed portion 71, the inclined surfaces 82 of the protruding portion 80 are provided. According to the shape of the protruding portions 80 in the present embodiment, in the absorbent body 1, formed are a low-SAP region A1 having a low basis weight of the SAP, a high-SAP region A2 having a high basis weight of the SAP, and an average-SAP region A3 having an average basis weight of the SAP, as illustrated in FIG. 5C.

As mentioned above, the basis weight of the SAP is adjusted only by providing the protruding portion 80 on the recessed portion 71, and this makes control easier and simplifies the device configuration compared to the case where the basis weight of the SAP is adjusted by adjusting a timing and a mechanism on a supply side of the SAP, for example. Further, it is possible to realize acceleration of manufacturing line, and it is possible to reliably adjust the basis weight of the SAP in a desired region of the absorbent body 1. The duct 23 can continuously supply a constant amount of the SAP, and therefore there is small affect on the other region in which a basis weight of the SAP is not adjusted. Accordingly, the SAP is evenly dispersed over the other region, making it possible to reliably ensure absorbent performance of the absorbent body 1.

Note that, a specific range of the angles θ1 and θ2 of the inclined surfaces 82 can be exemplified by 20 degrees to 60 degrees, and more preferably, 30 degrees to 45 degrees. The angle of repose of the superabsorbent polymer (SAP) is an angle of repose of the SAP before a supply of water during manufacturing. An angle of repose of the superabsorbent polymer (SAP) may be measured by a known method. For example, examples of the known method include a method for obtaining the angle by using a Sugihara-style repose angle measuring instrument.

In the device 10 in the present embodiment, the shaping die 70 including the recessed portions 71 is installed on the rotary drum 31. Thus, the cross section orthogonal to the ridge line 81 of the protruding portion 80 includes a cross section in which the bottom surface 71c of the recessed portion 71 is slightly curved. The bottom surface 71c in the cross section of FIG. 5A taken along line A-A in FIG. 3C is slightly curved, but the bottom surface 71c is illustrated to be horizontal for simplification of the drawing. In this case, in the "angle of the inclined surface" in the present invention may be defined as follows : in the cross section orthogonal to the ridge line 81 of the protruding portion 80, the angle of the inclined surface 82 with respect to a tangent line of the bottom surface 71c of the recessed portion 71 at the point where the bottom surface 71c intersects the inclined surfaces 82; or in the cross section of the bottom plate 73 before bending along the rotary drum 31 or in the cross section of the bottom plate 73 in which the bottom surface 71c of the recessed portion 71 is restored to a flat surface, the angle of the inclined surfaces 82 with respect to a surface parallel to the bottom surface 71c of the recessed portion 71.

In order to supply the pulp fiber 6 and the SAP to the recessed portion 71, the duct 23 is preferably provided to face at least the outer circumferential surface of a vertical upper half of the rotary drum 31. Furthermore, as illustrated in FIG. 2, the duct 23 is provided to face the top portion of the rotary drum 31 and the outer circumferential surface in the vicinity thereof, and therefore when the SAP is supplied from the duct 23, the bottom surface 71c of the recessed portion 71 is substantially horizontal and the angle of the inclined surfaces 82 with respect to the horizontal surface is greater than the angle of repose of the SAP. This makes the SAP more reliably roll down the inclined surface 82, making it possible to adjust the basis weight of the SAP.

In contrast, the pulp fiber 6 does not have a particulate shape like the SAP and thus has low flowability, and is deposited on the inclined surfaces 82 of the protruding portion 80 without rolling down the inclined surfaces 82. In particular, in the present embodiment, the suction force also acts on the inclined surfaces 82 of the protruding portion 80, and therefore the pulp fiber 6 is more reliably deposited on the inclined surfaces 82. Accordingly, even with the protruding portion 80 provided on the recessed portion 71, the basis weight of the pulp fiber 6 deposited on the inclined surfaces 82 is not affected.

In the manufacturing device 10 in the first embodiment, constant amounts of the pulp fiber 6 and the SAP are continuously supplied from the duct 23 to the recessed portions 71, and the suction force of the rotary drum 31 is constant. Furthermore, as illustrated in FIG. 4, the aperture ratio of the vent holes 76a is uniform in the air-permeable member 74 forming the bottom surface 71c of the recessed portion 71 and the protruding portions 80. Note that the aperture ratio of the vent holes 76a is a ratio of an area of the vent holes 76a per unit area. In the air-permeable member 74 in FIG. 4, the vent holes 76a having the same shape and the same size are arranged with fixed intervals and thus have a uniform aperture ratio.

Therefore, in the manufacturing device 10 in the first embodiment, the suction force acting on the bottom surface 71c of the recessed portion 71 and the protruding portions 80 can be constant, and the basis weight of the pulp fiber 6 can be uniform over the entire region of the absorbent core 2 shaped in the recessed portion 71. In other words, in a portion of the absorbent core 2 formed on the inclined surfaces 82 of the protruding portions 80, the basis weight of the SAP is lower than that in the other portion, but the basis weight of the pulp fiber 6 does not vary. In this way, providing the protruding portions 80 on the recessed portion 71 and adjusting the aperture ratio of the vent holes 76a of the air-permeable member 74 make it possible to individually adjust the basis weight of the pulp fiber 6 and the basis weight of the SAP.

The manufacturing device 10 in the first embodiment further includes the downstream embossing unit 60 (a compressing unit in the present invention) that compresses, in the thickness direction of the absorbent body 1, the low-SAP region A1, which is the portion of the absorbent body 1 formed by the inclined surfaces 82 of the protruding portions 80. In other words, the pair of linear compressed portions 4a are formed in the low-SAP region A1.

Compression is properly performed in the region having a small basis weight of the SAP, making it possible to more reliably form the pair of linear compressed portions 4a in the absorbent body 1. This can increase the rigidity of the absorbent body 1, making it possible to suppress a loss of the shape thereof. The pair of linear compressed portions 4a restricts movement of the pulp fiber 6 and the SAP, and this maintains the distribution of the pulp fiber 6 and the SAP, which has been adjusted using the protruding portions 80 and the aperture ratio of the air-permeable member 74 . The linear compressed portions 4a which are relatively strongly compressed form in the low-SAP region A1, and this makes possible to prevent the linear compressed portions 4a from excessively hardening due to compression of a large amount of the SAP. Consequently, an absorbent article can fit more comfortably.

In addition, the manufacturing device 10 in the first embodiment includes the upstream embossing unit 50 (a second compressing unit in the present invention) that compresses, in the thickness direction of the absorbent body 1, a portion of the absorbent body 1 including at least a part of the high-SAP region A2 and a part of the average-SAP region A3, which are formed using the bottom surface 71c of the recessed portion 71. Note that, the upstream embossing unit 50 in the present embodiment forms the spot-like compressed portions 4b over the entire region of the absorbent body 1, but the upstream embossing unit 50 is not limited this example. For example, the upstream embossing unit 50 may form the spot-like compressed portions 4b between the pair of linear compressed portions 4a. The present invention is not limited to embossing that performs compression in a discrete manner, and, for example, so-called pressing that uniformly compresses the entire surface of the absorbent body 1 may be performed.

In this way, it is possible to further increase the rigidity of the absorbent body 1. The pulp fiber 6 and the SAP become integrated state in the high-SAP region A2 and the average-SAP region A3, and movement of the pulp fiber 6 and the SAP is restricted, maintaining the distribution of the pulp fiber 6 and the SAP adjusted by the protruding portions 80 and the aperture ratio of the air-permeable member 74. Note that, the order of the upstream embossing unit 50 and the downstream embossing unit 60 may be reversed, and the manufacturing device of the absorbent body may include only one of them or may not include both of them.

Each of the protruding portions 80 in the present embodiment includes the two inclined surfaces 82 (an inclined surface and a second inclined surface in the present invention) on both sides of the ridge line 81. The two inclined surfaces 82 have both the angles θ1 and θ2 greater than an angle of repose and smaller than 90 degrees. Thus, the SAP can roll down to both sides in the direction orthogonal to the ridge line 81 of the protruding portion 80, and the high-SAP region A2 can be formed on both sides of the low-SAP region A1. The protruding portions 80 in the present embodiment are located in regions corresponding to the linear compressed portions 4b, and therefore the high-SAP region A2 is formed around the linear compressed portions 4a formed in the absorbent body 1, as illustrated in FIG. 5C.

Furthermore, the two inclined surfaces 82 of each protruding portion 80 in the present embodiment have different angles θ1 and θ2. Specifically, as illustrated in FIG. 5A, the angle θ1 of the inner inclined surface 83 is smaller than the angle θ2 of the outer inclined surface 84. Thus, in the direction orthogonal to the ridge line 81, the length L1 of the inner inclined surface 83 is larger than the length L2 of the outer inclined surface 84. And the planar area of the inner inclined surface 83 as viewed in the direction of deposition is greater than the planar area of the outer inclined surface 84. Therefore, the amount of the SAP reaching and rolling down on the inner inclined surface 83 is greater than the amount of the SAP reaching and rolling down on the outer inclined surface 84.

In this way, concerning the two inclined surfaces 82 disposed on both sides of the ridge line 81, in other words, the two inclined surfaces 82 having an identical height, differing the angles θ1 and θ2 of the two inclined surfaces 82 can make the basis weight of the SAP different between the high-SAP regions A2 in both sides of the ridge line 81. A smaller angle of the inclined surface 82 increases an increase amount of the SAP in the high-SAP region A2, and a greater angle of the inclined surface 82 reduces an increase amount of the SAP in the high-SAP region A2. Accordingly, adjusting the angles of the inclined surfaces 82 makes it possible to adjust an increase amount of the SAP in the high-SAP region A2.

In the present embodiment, the longitudinal direction and the width direction of the absorbent body 1 respectively correspond to the MD direction and the CD direction. Thus, the direction orthogonal to the ridge line 81 of the protruding portion 80 extending in the MD direction is a substantial CD direction close to the CD direction. In general, the widthwise central portion of the absorbent body 1 comes in contact with an excretory portion of a wearer and is a region receiving a large amount of excrement. Thus, the central portion in the width direction of the absorbent body 1 can have a great basis weight of the SAP by setting the angle θ1 of the inner inclined surface 83 in the substantial CD direction to be smaller than the angle θ2 of the outer inclined surface 84, making more amount of SAP roll down inwardly in substantially the CD direction. However, the present invention is not limited to that described above, and the two inclined surfaces 82 of the protruding portion 80 may have the equal angle.

As in a protruding portion 80 in a modified example illustrated in FIG. 6A, an inclined surface 82 on which the SAP rolls down may be provided on only one side in the direction orthogonal to the ridge line 81. And, on the other side, a cliff surface 85 having an angle θ3 of 90 degrees with respect to a surface 71d that is parallel to the bottom surface 71c of the recessed portion 71 may be provided. In this case, the high-SAP region A2 is formed on only one side of the low-SAP region A1 formed by the inclined surface 82, and the average-SAP region A3 is formed on the other side. In this way, the number of the high-SAP regions A2 can be adjusted by adjusting the number of the inclined surfaces 82 of the protruding portion 80.

The protruding portion 80 in the present embodiment has a mountain-shaped cross-section and does not have a flat surface between the inner inclined surface 83 and the outer inclined surface 84. Thus, the SAP can be prevented from being deposited in the center of the low-SAP region A1 which is formed by the inner inclined surface 83 and the outer inclined surface 84. In other words, concerning the portion of the absorbent body 1 formed by the protruding portion 80, the entire region thereof can be the low-SAP region A1. For example, since the protruding portions 80 in the present embodiment are located in regions corresponding to the linear compressed portions 4a, preventing deposition of the SAP on the portions of the absorbent body 1 formed by the protruding portions 80 makes it possible to prevent the linear compressed portions 4a from excessively hardening due to compression of the SAP.

Note that, a ridge line portion of the protruding portion 80 may have a round shape (R shape). In other words, the inner inclined surface 83 and the outer inclined surface 84 of the protruding portion 80 may be connected to each other to form a curved surface protruding outward. Also in this case, the SAP rolls down from the curved ridge line portion, making it possible to prevent the SAP from being deposited on the portion of the absorbent body 1 formed by the protruding portion 80. However, the present invention is not limited to this. As illustrated in FIG. 6B, two inclined surfaces 82 of a protruding portion 80 may be connected to each other with a flat surface 86 to form the average-SAP region A3 at the center of the low-SAP region A1 formed by the two inclined surfaces 82.

In the manufacturing device 10 in the first embodiment, the supply unit 20 supplies the pulp fiber 6 and the SAP to the recessed portion 71 moving in the circumferential direction (predetermined direction) of the rotary drum 31. The SAP has a higher degree of flowability than that of the pulp fiber 6, and therefore the SAP is more likely to move upstream in the MD direction, which is the circumferential direction of the rotary drum 31, due to inertial force of the rotation of the rotary drum 31. In the present embodiment, the longitudinal direction of the absorbent body 1 corresponds to the MD direction, and the protruding portions 80 extend in the MD direction.

It is assumed herein that the ridge line 81 (inclined surfaces 82) of each protruding portion 80 extends straightly along the MD direction. In this case, the high-SAP region A2 extending straightly in the MD direction is formed at a certain part in the CD direction. Thus, the SAP in the high-SAP region A2 successively moves upstream in the MD direction, and the SAP is concentrated at a certain part of the recessed portion 71 in the CD direction being located upstream in the MD direction.

In contrast, the ridge line 81 of the protruding portion 80 in the present embodiment includes a curved portion (for example, 81a illustrated in FIG. 4 and the like) curved with respect to the MD direction. Accordingly, as illustrated in FIG. 5C, the high-SAP region A2 formed in the absorbent body 1 is curved with respect to the MD direction (longitudinal direction), and the high-SAP region A2 is formed in a different position in the CD direction (width direction). Thus, even when the SAP moves upstream in the MD direction due to inertial force, the SAP is dispersed through the CD direction, making it possible to suppress concentration of the SAP at a certain part of the recessed portion 71 in the CD direction. That the ridge line 81 of the protruding portion 81 is curved with respect to the MD direction makes the SAP moving upstream in the MD direction be intercepted by the inclined surface 82.

For example, an inclined surface 84e (see FIG. 4) located at an end portion of the protruding portion 80 is greatly inclined outwardly in the CD direction. In the MD direction, the side on which the inclined surface 84e is inclined outwardly in the CD direction may be set to be the upstream side in the MD direction. In this way, more amount of SAP moving upstream in the MD direction can be intercepted by the inclined surface 84e. However, the present invention is not limited to that described above, and a part or the whole of the ridge line of the protruding portion 80 may extend straightly in the MD direction.

### Second Embodiment

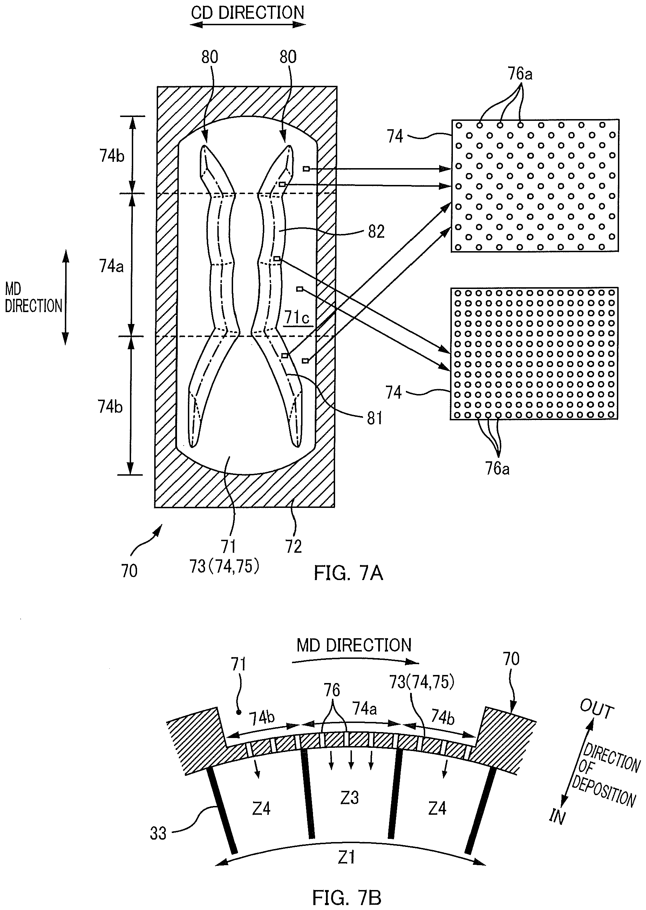

FIGS. 7A and 7B are diagrams illustrating a manufacturing device of an absorbent body 1 according to a second embodiment. FIG. 7A is a diagram illustrating vent holes 76a of an air-permeable member 74. FIG. 7B is a schematic cross-sectional view illustrating a difference of negative pressure in space (first zone Z1) on an inner circumferential side of a rotary drum 31. Note that, protruding portions 80 included in a recessed portion 71 is omitted from FIG. 7B.

In the manufacturing device in the second embodiment, similarly to the first embodiment, a pulp fiber 6 and SAP are deposited on the recessed portion 71 by suction, and a constant amount of the pulp fiber 6 and the SAP is continuously supplied from a duct 23. Then, the manufacturing device in the second embodiment adjusts the basis weight of the pulp fiber 6 of a portion of the absorbent body 1 different from linear compressed portions 4a while keeping the basis weight of the SAP of the linear compressed portions 4a low similarly to the first embodiment. In other words, the basis weight of the SAP and the basis weight of the pulp fiber 6 are individually adjusted. Hereinafter, description is made by taking, as an example, a case where a basis weight of the pulp fiber 6 is greater at a central portion in the longitudinal direction of an absorbent core 2 than that at both end portions.

In order to manufacture the absorbent body 1 described above, it is preferable that the aperture ratio of the vent holes 76a is different depending on a portion of the air-permeable member 74, in other words, a portion of a bottom portion 71b of the recessed portion 71, as illustrated in FIG. 7A for example. Specifically, the aperture ratio of the vent holes 76a preferably increases by setting an interval between the vent holes 76a to be smaller at the MD-direction central portion 74a of the air-permeable member 74 than at both MD-direction end portions 74b of the air-permeable member 74. In this way, the per-unit-area suction force acting on the MD-direction central portion 74a of the air-permeable member 74 can be stronger than the per-unit-area suction force acting on the both MD-direction end portions 74b of the air-permeable member 74. Accordingly, a larger amount of pulp fiber 6 are deposited on the MD-direction central portion 74a of the air-permeable member 74.

Further, as illustrated in FIG. 7B, the negative pressure in a space communicating with the vent holes 76a, in other words the negative pressure in the first zone Z1 of the rotary drum 31 may be different depending on a portion of the air-permeable member 74, in other words, a portion of the bottom portion 71b of the recessed portion 71. Specifically, the first zone Z1 of the rotary drum 31 is partitioned by partition walls 33, and the negative pressure in a third zone Z3 corresponding to the MD-direction central portion 74a of the air-permeable member 74 is higher than the negative pressure in fourth zones Z4 corresponding to the both MD-direction end portions 74b of the air-permeable member 74. For this purpose, the following methods may be used: , for example, increasing the number of revolutions of a suction fan (not shown) that generates negative pressure in the third zone Z3; increasing the number of suction fans; and increasing the size of the suction fan. This can makes the per-unit-area suction force acting on the MD-direction central portion 74a of the air-permeable member 74 stronger than the per-unit-area suction force acting on the both MD-direction end portions 74b of the air-permeable member 74. Accordingly, a larger amount of pulp fiber 6 are deposited on the MD-direction central portion 74a of the air-permeable member 74.

In contrast, the SAP has a weight greater than that of the pulp fiber 6 and is less affected by the suction force acting on the air-permeable member 74. And the basis weight of the SAP is less likely to fluctuate by a difference of the suction forces acting on the air-permeable member 74. Thus, as in FIGS. 7A or 7B, or as in the combined configuration of FIGS. 7A and 7B, the protruding portions 80 may be provided on the recessed portion 71 similarly to the first embodiment while the suction forces acting on the air-permeable member 74 are adjusted. In this way, it is possible to manufacture the absorbent body 1 described above in which the basis weight of the SAP of the linear compressed portions 4a is lower than that in the other portion and in which the basis weight of the pulp fiber 6 at the central portion in the longitudinal direction is greater than that at the both end portions.

In general, a per-unit-time supply amount of the pulp fiber 6 and the SAP from the duct 23 changes according to a change in speed of the manufacturing line, in other words, a change in rotation speed of the rotary drum 31. Accordingly, it is preferable that the suction force on the first zone Z1 side of the rotary drum 31 change according to a change in rotation speed of the rotary drum 31. For example, increase of the speed of the rotary drum 31 increase the supply amount of the pulp fiber 6 and the SAP, and therefore it is preferable that the suction force in the first zone Z1 increases. In contrast, decrease of the speed of the rotary drum 31 decreases the supply amount of the pulp fiber 6 and the SAP, and therefore it is preferable that the suction force in the first zone Z1 decreases. Thereby, a change in rotation speed of the rotary drum 31 becomes less likely to affect on a distribution of the pulp fiber 6 and the SAP adjusted by the aperture ratio of the air-permeable member 74, the protruding portions 80, and the like. This makes possible to stably adjust the basis weights of the pulp fiber 6 and the SAP in a desired region of the absorbent body 1.

### Third Embodiment

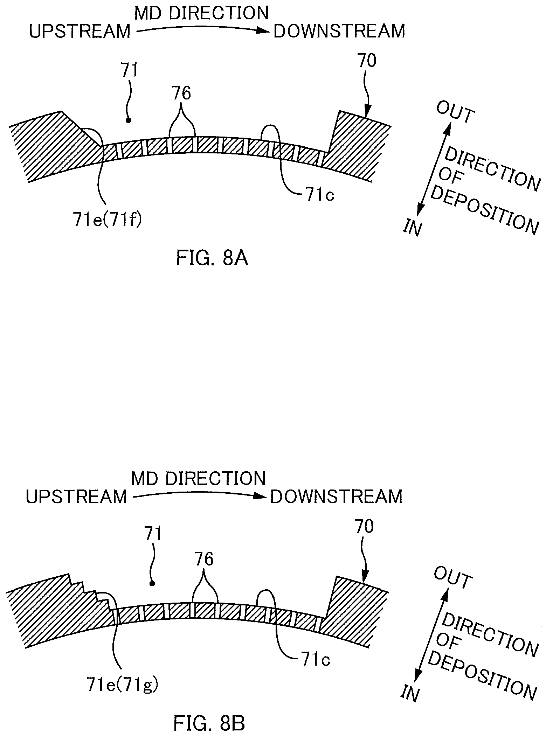

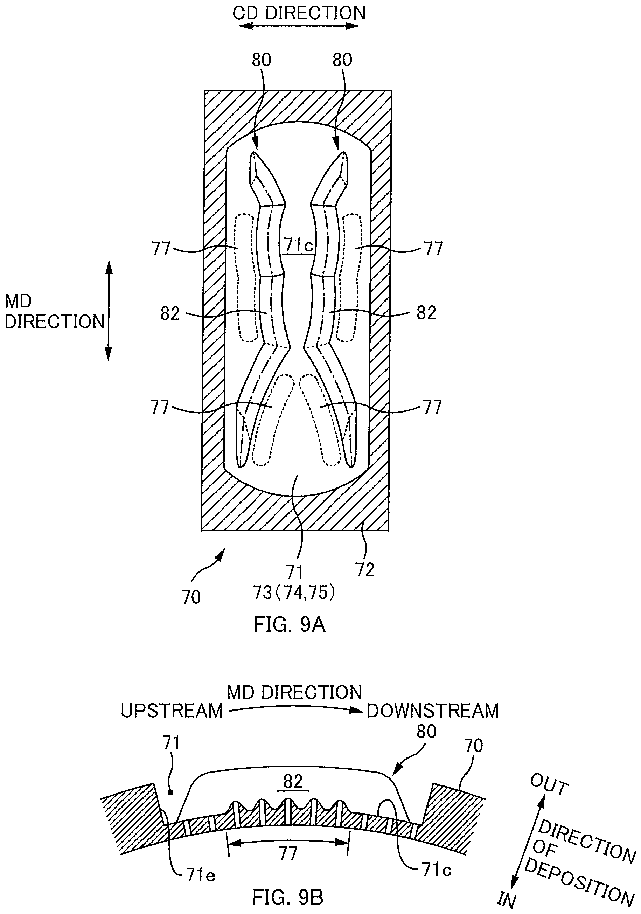

FIGS. 8A and 8B are schematic cross-sectional views illustrating a recessed portion 71 of a manufacturing device according to a third embodiment. Note that, protruding portions 80 are omitted from FIG. 8. FIGS. 9A and 9B are diagrams illustrating a different recessed portion 71 of the manufacturing device according to the third embodiment. FIG. 9A is a schematic plan view illustrating a position in which a concave/convex portion 77 formed on a bottom surface 71c of the recessed portion 71 is formed. FIG. 9B is a schematic cross-sectional view of the recessed portion 71.

In the manufacturing device in the third embodiment, similarly to the first embodiment, a supply unit 20 supplies pulp fiber 6 and SAP to the recessed portion 71 moving in the circumferential direction (predetermined direction) of a rotary drum 30. As described above, the SAP is more likely to move upstream in the MD direction (the circumferential direction of the rotary drum 31) due to inertial force of the rotation of the rotary drum 31. Thus, the SAP which has moved is more likely to collide with a side surface 71e on the upstream side of the recessed portion 71 and be deposited on the place.

For example, when the protruding portions 80 provided on the recessed portion 70 elongate in the MD direction as illustrated in FIG. 3C, the high-SAP region A2 formed by the SAP rolling down the inclined surfaces 82 of the protruding portions 80 also elongates in the MD direction. Accordingly, the SAP in the high-SAP region A2 successively moves upstream in the MD direction, and a large amount of SAP are concentrated near the side surface 71e on the upstream side of the recessed portion 71. This makes the SAP be more likely to overflow from an end portion of the absorbent body 1.

Thus, the side surface 71e of the recessed portion 71 on the upstream side in the MD direction may be an inclined surface 71f (FIG. 8A) inclined toward the upstream side in the MD direction outwardly from a bottom surface 71c of the recessed portion 71 (to the supply unit 20 side in the direction of deposition) or may be a step surface 71g (FIG. 8B) having a step and inclined toward the upstream side in the MD direction outwardly from the bottom surface 71c.

In this way, the SAP moving upstream in the MD direction is more likely to move along the inclined surface 71f or the step surface 71g due to momentum, jumping outside the recessed portion 71 (upstream side). Further, the pulp fiber 6 is successively deposited on the bottom surface 71c of the recessed portion 71, and the pulp fiber 6 is also deposited on the inclined surface 71f or the step surface 71g. Accordingly, in particular, the SAP supplied at a relatively later timing, in other words, the SAP that has moved above the deposited pulp fiber 6 is more likely to jump outside the recessed portion 71. This makes it possible to suppress the concentration of the SAP near the side surface 71e on the upstream side of the recessed portion 71.

As illustrated in FIG. 9, the concave/convex portion 77 having a height (in the direction of deposition) lower than the inclined surfaces 82 of the protruding portions 80 and elongating in the MD direction may be provided on a portion of the bottom surface 71c of the recessed portion 71 around the inclined surfaces 82 of the protruding portions 80. In other words, the concave/convex portion 77 is provided in the high-SAP region A2.

The pulp fiber 6 is deposited in conformance with the shape of the bottom surface 71c of the recessed portion 71. Thus, the pulp fiber 6 is also deposited in an undulating manner on the concave/convex portion 77 of the bottom surface 71c. Accordingly, even when the SAP reaching the concave/convex portion 77 tries to move upstream in the MD direction by inertial force of the rotary drum 31, the SAP is intercepted by a protruding portion of the pulp fiber 6 deposited unevenly. In this way, the movement of the SAP to move upstream is restricted by providing the concave/convex portion 77 on the bottom surface 71c of the recessed portion 71, making it possible to suppress the concentration of the SAP near the side surface 71e on the upstream side of the recessed portion 71.

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof.

### [Reference Signs List]

1: absorbent body;
2: absorbent core;
3: core wrapping sheet;
4: compressed portion;
4a: linear compressed portion;
4b: spot-like compressed portion;
5: pulp sheet;
6: pulp fiber (liquid absorbent fiber);
sap: super absorbent polymer;
10: manufacturing device of absorbent body;
20: supply unit;
21: pulverizer;
22: sap supply tube;
23: duct;
30: shaping unit;
31: rotary drum;
32: partition wall;
33: partition wall;
Z1: first zone (space communicating with vent holes);
Z2: second zone;
40: suction belt conveyer;
41: transport belt;
42: suction mechanism;
50: upstream embossing unit (second compressing unit);
51: roll;
60: downstream embossing unit (compressing unit);
61: roll;
70: shaping die;
71: recessed portion;
71a: side portion;
71b: bottom portion;
71c: bottom surface;
71d: surface parallel to bottom surface;
71e: side surface;
71f: inclined surface;
71g: step surface;
72: side plate;
73: bottom plate;
74: air-permeable member;
75: reinforcing member;
76: vent hole;
76a: vent hole;
76b: vent hole (second vent hole);
76c: vent hole (second vent hole);
77: uneven potion;
80: protruding portion;
81: ridge line;
81a: curved portion;
82: inclined surface (inclined surface and second inclined surface);
83: inner inclined surface;
84: outer inclined surface.

## Claims

1. A device for manufacturing an absorbent body including a liquid absorbent fiber and a superabsorbent polymer, the device comprising:
a shaping unit
that includes a recessed portion, and
that makes the liquid absorbent fiber and the superabsorbent polymer to be deposited in the recessed portion, shaping the absorbent body,
the recessed portion including a protruding portion,
the protruding portion protruding from a bottom surface and including an inclined surface; and
a supply unit that supplies the liquid absorbent fiber and the superabsorbent polymer to the recessed portion,
in a cross section orthogonal to a ridge line of the protruding portion,
an angle of the inclined surface with respect to a surface parallel to the bottom surface being greater than an angle of repose of the superabsorbent polymer and smaller than 90 degrees.

2. The device for manufacturing an absorbent body according to claim 1, wherein
the protruding portion includes a second inclined surface, and in the cross section,
an angle of the second inclined surface with respect to a surface parallel to the bottom surface is greater than the angle of repose of the superabsorbent polymer and smaller than 90 degrees, and
the angle of the second inclined surface is different from the angle of the inclined surface.

3. The device for manufacturing an absorbent body according to claim 1 or 2, wherein
the protruding portion includes a second inclined surface, and
a flat surface is not provided between the inclined surface and the second inclined surface.

4. The device for manufacturing an absorbent body according to any one of claims 1 to 3, wherein
the supply unit supplies the liquid absorbent fiber and the superabsorbent polymer to the recessed portion moving in a predetermined direction, and
a ridge line of the protruding portion includes a curved portion curved with respect to the predetermined direction.

5. The device for manufacturing an absorbent body according to any one of claims 1 to 4, wherein
the shaping unit makes the liquid absorbent fiber and the superabsorbent polymer to be deposited in the recessed portion by suction,
the bottom surface and the protruding portion are formed of an air-permeable member having a plurality of vent holes, and
the vent holes in the air-permeable member have a uniform aperture ratio.

6. The device for manufacturing an absorbent body according to any one of claims 1 to 4, wherein
the shaping unit makes the liquid absorbent fiber and the superabsorbent polymer to be deposited in the recessed portion by suction,
the bottom surface and the protruding portion are formed of an air-permeable member having a plurality of vent holes, and
an aperture ratio of the vent holes is different depending on a portion of the air-permeable member.

7. The device for manufacturing an absorbent body according to claim 5 or 6, wherein
a reinforcing member along the air-permeable member is provided inside the air-permeable member,
the reinforcing member includes a plurality of second vent holes that is larger than the vent holes, and
the second vent hole disposed in a portion of the reinforcing member corresponding to the inclined surface are smaller than the second vent hole disposed in a portion of the reinforcing member corresponding to the bottom surface.

8. The device for manufacturing an absorbent body according to any one of claims 5 to 7, wherein
a negative pressure in a space communicating with the vent hole is different depending on a portion of the air-permeable member.

9. The device for manufacturing an absorbent body according to any one of claims 1 to 8, wherein
the device further comprises a compressing unit that compresses, in a thickness direction of the absorbent body, a portion of the absorbent body formed using the inclined surface.

10. The device for manufacturing an absorbent body according to any one of claims 1 to 9, wherein
the device further comprises a second compressing unit that compresses, in the thickness direction of the absorbent body, a portion of the absorbent body including at least a part of a portion formed of the bottom surface.

11. The device for manufacturing an absorbent body according to any one of claims 1 to 10, wherein
the supply unit supplies the liquid absorbent fiber and the superabsorbent polymer to the recessed portion moving in a predetermined direction, and
a side surface of the recessed portion on an upstream side in the predetermined direction is an inclined surface or a step surface having a step,
the inclined surface and the step surface being inclined toward the upstream side outwardly from the bottom surface.

12. The device for manufacturing an absorbent body according to any one of claims 1 to 11, wherein
the supply unit supplies the liquid absorbent fiber and the superabsorbent polymer to the recessed portion moving in a predetermined direction, and
a concave/convex portion having a height lower than the inclined surface and elongating in the predetermined direction is provided on a portion of the bottom surface around the inclined surface.

13. A method for manufacturing an absorbent body including a liquid absorbent fiber and a superabsorbent polymer, the method comprising:
supplying the liquid absorbent fiber and the superabsorbent polymer to a recessed portion that is for shaping the absorbent body; and
shaping the absorbent body by depositing the liquid absorbent fiber and the superabsorbent polymer in the recessed portion,
the recessed portion including a protruding portion,
the protruding portion protruding from a bottom surface and including an inclined surface,
in a section orthogonal to a ridge line of the protruding portion,
an angle of the inclined surface with respect to a surface parallel to the bottom surface being greater than an angle of repose of the superabsorbent polymer and smaller than 90 degrees.
